# EUROPÄISCHE PATENTSCHRIFT

(11) **EP 1 820 018 B1**
(45) Veröffentlichungstag und Bekanntmachung des Hinweises auf die Patenterteilung: **21.09.2011**
(21) Anmeldenummer: 05824361.9
(22) Anmeldetag: 06.12.2005
(51) Int. Cl.: G01N 33/52

(54) **TESTELEMENT MIT NANOFASERN**
TEST ELEMENT COMPRISING NANOFIBRES
ELEMENT D'ESSAI A NANOFIBRES

(30) Priorität: 07.12.2004 DE 102004058924
(43) Veröffentlichungstag der Anmeldung: 22.08.2007
(73) Patentinhaber: Roche Diagnostics GmbH, 68305 Mannheim (DE); F.Hoffmann-La Roche AG, 4070 Basel (CH)
(72) Erfinder: HARTTIG, Herbert, 67434 Neustadt (DE)
(74) Vertreter: Weiss, Wolfgang
(86) Internationale Anmeldenummer: PCT/EP2005/013066
(87) Internationale Veröffentlichungsnummer: WO 2006/061189

(56) Entgegenhaltungen:
- EP-A1- 1 564 315
- WO-A-2004/038073
- WO-A-2004/099068
- WO-A1-02/40242
- LEE, S.-H., ET AL.: 'Design, Synthesis and Electrospinning of a Novel Fluorescent Polymer for Optical Sensor Applications' MATERIALS RESEARCH SOCIETY SYMPOSIUM PROCEEDINGS Bd. 708, 2002, Seiten 10.45.1 - 10.45.6 Gefunden im Internet: <URL:http://www.mrs.org/s_mrs/bin.asp?CID=2 516&DID=56891&DOC=FILE.PDF>
- SUN JONG KWOUN ET AL: "A novel polymer nanofiber interface for chemical sensor applications", FREQUENCY CONTROL SYMPOSIUM AND EXHIBITION, 2000. PROCEEDINGS OF THE 2 000 IEEE/EIA INTERNATIONAL 7-9 JUNE 2000, PISCATAWAY, NJ, USA,IEEE, 1 January 2000 (2000-01-01), pages 52-57, XP010525523, ISBN: 978-0-7803-5838-6
- FONG H ET AL: "Beaded nanofibers formed during electrospinning", POLYMER, ELSEVIER SCIENCE PUBLISHERS B.V, GB LNKD- DOI:10.1016/S0032-3861(99)00068-3, vol. 40, no. 16, 1 July 1999 (1999-07-01), pages 4585-4592, XP004205846, ISSN: 0032-3861
- ZHENG-MING HUANG ET AL: "A review on polymer nanofibers by electrospinning and their applications in nanocomposites", COMPOSITES SCIENCE AND TECHNOLOGY, ELSEVIER, UK LNKD- DOI:10.1016/S0266-3538(03)00178-7, vol. 63, no. 15, 2 July 2003 (2003-07-02), pages 2223-2253, XP002516036, ISSN: 0266-3538 [retrieved on 2003-07-02]
- ZONG X ET AL: "Structure and process relationship of electrospun bioabsorbable nanofiber membranes", POLYMER, ELSEVIER SCIENCE PUBLISHERS B.V, GB LNKD- DOI:10.1016/S0032-3861(02)00275-6, vol. 43, no. 16, 1 July 2002 (2002-07-01), pages 4403-4412, XP004373517, ISSN: 0032-3861
- LEE, S.-H., KU, B.-C., WANG, X., SAMUELSON, L. A., KUMAR, J.: "Design, Synthesis and Electrospinning of a Novel Fluorescent Polymer for Optical Sensor Applications" MATERIALS RESEARCH SOCIETY SYMPOSIUM PROCEEDINGS, Bd. 708, 2002, Seiten BB10.45.1-BB10.45.6,
- XP010525523

## Beschreibung

Gegenstand der Erfindung sind Testelemente, insbesondere diagnostische Testelemente, zur Ermittlung der Anwesenheit oder Konzentration von biologischen, medizinischen oder biologisch oder medizinisch wirksamen Substanzen, inklusive Nukleinsäuren, Proteinen, Viren, Mikroorganismen und Zellen, wie sie in den Ansprüchen definiert sind.

Diagnostische Testelemente, insbesondere Teststreifen, enthalten dem Stand der Technik nach vielfältige, auf Fasern basierende Materialien. Insbesondere zu erwähnen sind Papiere oder Vliese. Speziell Vliese werden genutzt, um die Abtrennung von unerwünschten Probenbestandteilen durchzuführen. Als Beispiel wird verwiesen auf die Blutabtrennungsvliese bei NW-Glucosetesten oder bei den Teststreifen aus dem Reflotron^{®} System. Die Fasern, die bei Papieren oder Vliesen des Standes der Technik verwendet werden, sind gekennzeichnet durch Durchmesser zwischen ca. 5 µm und 200 µm.

Nanofasern sind im Prinzip bekannt und seit ca. 1930 Stand der Technik. Sie werden erzeugt durch das so genannte Elektrospinnverfahren, bei dem durch Anlegen von hoher Spannung im Bereich von 10-55 kV an einen Tropfen einer Polymerlösung oder Polymerschmelze eine dünne Faser erzeugt wird (Formhals, A., US-Patente 1,975,504 (1934), 2,160,962 (1939, 2,187,306 (1940)).

Nanofasern zeichnen sich durch extrem geringe Durchmesser aus. Je nach Material sind die Fasern mit Durchmessern von 10-2000 nm erhältlich. Teilweise werden verzweigte Fasern erhalten oder Fasern, die mehr oder weniger große, mehr oder weniger häufige Polymerperlen (Beads) auf dem Faden enthalten. Wichtige Einflussgrößen sind dem Fachmann bekannt oder der einschlägigen Literatur (z.B. Li und Xia, Adv. Mater. 16 (2004), 1151-1170) zu entnehmen.

Die Verwendung von Nanofasern in Medizinprodukten ist in US 2003/ 0171053 beschrieben. Dabei handelt es sich um eine medizinische Vorrichtung, die mit einer Nanofaserschicht umhüllt ist, um die Bioverträglichkeit zu verbessern. Weitere Publikationen beziehen sich auf eine Gehimsonde, die zur Verbesserung der Biokompatibilität und Messstabilität mit Polylactid-Nanofasern belegt ist (z.B. US 2002/0106496 oder DiPaolo et al., Proc. 2nd Joint EMBS/BMES Conf. Houston TX, USA, 23-23, Oktober 2002).

US 2003/0217928 offenbart ein Verfahren zur Elektrosynthese von Nanostrukturen, die zur Detektion eines Analyten, auch innerhalb eines elektrisch leitfähigen Arrays, eingesetzt werden können.

WO 02/40242 beschreibt ein Verfahren zur Herstellung von Produkten für den medizinischen und zellbiologischen Einsatz, z.B. Stents, durch Elektrospinnen von Nanofasern auf Basis von Collagen.

WO 03/026532 bescheibt die Verwendung von Nanofasern für medizinische Geräte, beispielsweise Ballons, Katheter, Filter und Stents. Es findet sich keinerlei Hinweis auf die Verwendung von Nanofasern in diagnostischen Testelementen.

WO 03/087443 offenbart ein Verfahren zum Aufbringen von Nanofasern auf einen Gegenstand, beispielsweise medizinische Geräte, wie Stents, oder Vorrichtungen zur kontrollierten Freisetzung von Arzneimitteln.

Feng et al. (Angew. Chem. Int. Ed. 42 (2003), 800-802) und Feng et al. (Angew. Chem. Int. Ed. 41 (2002), 1221-1223) beschreiben die Herstellung superhydrophober Oberflächen aus kurzen Nanofasern.

WO 2004/099068 A2 betrifft Nanofasern, Nanofasern enthaltende Strukturen sowie deren Verwendung im Rahmen analytischer Verfahren. Im Einzelnen werden Trägerelemente beschrieben, welche mindestens eine erste Oberfläche, eine Vielzahl von an die erste Oberfläche gebundenen Nanofasern sowie eine oder mehrere spezifische Einheiten, welche an mindestens eine der Nanofasern gebunden sind, umfassen. Die hierbei verwendeten Nanofasern besitzen eine Länge von etwa 2 µm bis etwa 1 mm sowie einen Durchmesser von etwa 5 nm bis etwa 1 µm und umfassen verschiedene anorganische und/oder organische Materialien, wobei durch geeignete Derivatisierung je nach Bedarf hydrophile oder hydrophobe Nanofasern bzw. entsprechend oberflächenmodifizierte Träger bereitgestellt werden können.

EP 1 564 315 A1 offenbart Aggregate von Nanofasern, welche aus einem thermoplastischen Polymer bestehen und in Bezug auf ihre Feinheit sehr geringe Unterschiede aufweisen. Die Herstellung der Nanofasern, welche u.a. als Blutfilter in medizinischen Vorrichtungen eingesetzt werden können, erfolgt durch Schmelzspinnen einer aus mehreren Polymeren erhaltenen Polymerlegierung unter Anwendung von Zieh- und Wärmebehandlungsprozessen.

Die Verwendung von Fasern üblichen Durchmessers in Testelementen für diagnostische Anwendungen hat spezifische Nachteile. Dies betrifft insbesondere die Separation von Blutzellen. Hier ergibt sich eine relativ grobe, uneinheitliche Porosität. Durch die großen Poren werden Blutzellen entweder nicht zurückgehalten oder sie werden im Inneren einer Gewebelage oder eines Vlieses zurückgehalten. Dabei kommt es, bedingt durch die großen Poren, zu einer Lyse infolge von hoher Kapillarität oder durch Verletzung der Membran von roten Blutkörperchen an scharfen Ecken und Kanten, besonders bei Glasfaservliesen. Nachteilig bei Vliesen oder Geweben nach dem Stand der Technik ist auch, dass diese Materialien relativ dick werden und entsprechend große Volumina an Flüssigkeit in den Faserzwischenräumen zurückhalten. Gerade beim Entwicklungstrend zu immer kleineren Probenvolumina ist dies äußerst störend.

Gebilde aus hydrophoben konventionellen Fasern können als Flüssigkeitssperre dienen. Beispiele dafür sind unter dem Handelsnamen Tyvek^{®} bekannt. Nachteilig an diesen Gebilden ist jedoch, dass eine wässrige Lösung, die mit diesen Geweben in Kontakt kommt, nicht von der Oberfläche abperlt, sondern auf der Oberfläche verbleibt, wiewohl sie nicht in der Lage ist, in die Poren einzudringen.

Aufgabe der vorliegenden Erfindung war die Bereitstellung von Testelementen, mit denen die Nachteile des Standes der Technik zumindest teilweise beseitigt werden. Diese Aufgabe wird gelöst durch Testelemente zum Nachweis eines Analyten, wie sie in den Ansprüchen definiert sind.

Die Erfindung betrifft die Verwendung von Nanofasern bei der Herstellung von Testelementen, z.B. Teststreifen, Arrays oder Sensoren. Als Nanofasern im Sinne der vorliegenden Erfindung sind dabei elektrogesponnene und beispielsweise kontinuierliche Fasern zu verstehen. Die Fasern haben vorzugsweise einen Durchmesser von 10-500 nm. Elektrogesponnene bzw. kontinuierliche Nanofasern weisen für die Anwendung in Testelementen eine Länge von ≥ 2 mm auf. Dies steht im Gegensatz zu den bei Feng et al. (2002), supra und Feng et al. (2003), supra beschriebenen kurzen Nanofasern.

Die Nanofasern im Sinne der vorliegenden Erfindung können hydrophile Nanofasern, hydrophobe Nanofasern und Gemische davon sein.

Die Nanofasern werden aus Polymeren durch ein Elektrospinnverfahren hergestellt. Geeignete Verfahren sind in den zuvor genannten Dokumenten des Standes der Technik offenbart. Beispiele für geeignete Polymere sind organische Polymere, z.B. Polyolefine, wie etwa Polyethylen, Polypropylen, Cycloolefinpolymere, wie etwa Topas^{®}, Polypenten oder Copolymere davon, fluorierte oder teilfluorierte Polymere, wie Polytetrafluorethylen oder andere, Polyester, wie etwa Polyterephthalat, Polyamide, wie etwa Poly-εcaprolactam, Polyurethane, Polyaromaten, wie etwa Poly[p-xylylen] und Derivate davon, Polyvinylalkohole, Polyvinylamine, Polyethylenimine, Polyalkylenoxide, wie etwa Polyethylenoxide bzw. Kombinationen oder Copolymere davon. Des Weiteren können auch anorganische Nanofasern, wie etwa Nanofasern auf Basis von Oxiden, wie Silikaten, z.B Glas, wie Silikat-, Alkalisilikat- Quarz- oder Wasserglas, oder Nanofasern auf Basis von Metallalkoxy-Kondensaten oder Kombinationen davon verwendet werden. Auch Kombinationen organischer und anorganischer Nanofasern können eingesetzt werden.

Die Nanofasern als Bestandteile von analytischen Testelementen liegen vorzugsweise in Form von Vliesen, Geweben, Membranen, Schichten oder Kombinationen davon vor. Die Herstellung solcher Materialien kann wie zuvor erwähnt durch Elektrospinnen von Polymeren aus Lösung oder aus der Schmelze erfolgen.

Die Herstellung eines solchen Nanofasermaterials kann so erfolgen, dass die Fasern ungeordnet abgelegt werden. Es ist aber auch möglich, die Fasern mehr oder weniger geordnet abzulegen, um isotrope oder anisotrope Effekte zu erreichen. Die Materialeigenschaften können sowohl durch die Wahl des Materials als auch durch die Wahl der Faserdurchmesser als auch durch die Wahl der Faserdichte und der Spinnparameter in weiten Grenzen beeinflusst werden.

Die Applikation eines solchen Nanofasermaterials auf ein Testelement, z.B. einen Teststreifen, kann dadurch erfolgen, dass die Fasern einfach auf die Oberfläche aufgesponnen werden. Sie können auch aufkalandriert oder appliziert werden durch Aufbringen auf auf eine Kleberschicht, wie z.B. einen Acrylatkleber, einen Haftkleber oder ein Klebeband. Es ist auch möglich, das Trägermaterial durch Lösungsmittel anzulösen und die Fasern auf das gequollene Material abzulegen, genauso wie es möglich ist, durch geeignete Lösungsmittel bei der Herstellung der Fasern einen Anlöseeffekt auf der Oberfläche des Trägers zu erreichen, welcher dann infolge, nach Verdampfen des Lösungsmittels, zu einer festen Verbindung zwischen den Nanofasern und der Oberfläche führt. Hier bei werden die Bedingungen so gewählt, dass Nanofasern mit Beads gebildet werden. Es ist aber auch leicht möglich, diese Fasern mit anderen Fasern aus einer weiteren Düse zu vermischen oder nach Applikation einer ersten Schicht mit Beads, die besonders gute Verbindungen zum Trägermaterial ergeben, eine weitere Schicht mit Fasern anderer Gestalt und Dicke oder/und Material aufzubringen.

Das Testelement, das die Nanofasern enthält, kann beispielsweise ein Teststreifen, ein Array oder ein Sensor, z.B. ein elektrochemischer Sensor, sein. Die Nanofasern sind dabei auf poröse oder nicht-poröse Materialien des Testelements aufgebracht.

In einer bevorzugten Ausführungsform enthält das Testelement einen Teststreifen, der mindestens ein poröses Trägermaterial, beispielsweise in Form eines Papiers, eines Vlieses oder/und einer Membran, enthält. Dabei können Nanofasern zumindest auf eine Oberfläche eines solchen porösen Trägermaterials aufgebracht werden.

Durch Ablage der Nanofasern auf ein konventionelles Papier oder Vlies oder eine Membran kann die Oberfläche dieses Trägermaterials so modifiziert werden, dass eine wesentlich feinere Porengröße an der belegten Oberseite erreicht wird. Dadurch sind völlig andere, wesentlich verbesserte Filtereigenschaften erreichbar. Ein solcherart modifiziertes Material kann in an sich bekannter Weise durch Kleben oder Laminieren o.ä. auf einen Teststreifen gebracht werden.

Beispielsweise können Nanofasern in Filterelementen zur Abtrennung von partikulären Bestandteilen aus einer Probe verwendet werden. In einer bevorzugten Ausführungsform ist das Filterelement ein Element zur Abtrennung von Blutzellen, vorzugsweise Erythrozyten.

Dabei ist es möglich, durch Verwendung von Nanofasern in Blutabtrennungsvliesen die Neigung von Hämolyse zu unterbinden, da die feinen Fasern die Membran von Erythrozyten unterstützen und es nicht zum Zerreißen der Erythrozyten-Membran infolge von Kapillaraktivität kommt. Es ist auch praktisch ausgeschlossen, dass die wenigen feinen Faserenden von Nanofasern in der Lage sind, die Membran von Erythrozyten zu beschädigen und dadurch Hämolyse zu verursachen. Bei Verwendung eines Vlieses aus Nanofasern ist es möglich, ein sehr dünnes Vlies, z.B. mit einer Dicke von 0,02 µm bis 50 µm, bevorzugt 0,05 µm bis 5 µm, besonders bevorzugt 0,08 µm bis 2 µm, mit hoher Filtereffektivität bereitzustellen, welches dann auch in der Lage ist, sehr geringe Blutvolumina zu verarbeiten und selbst nur eine sehr geringe Rückhaltung besitzt. Bei dieser Anwendung werden bevorzugt hydrophile Polymere, wie etwa Polyamide, Polyurethane, Polyvinylalkohole, Polyvinylamine, Polyethylenimine, Polyethylenglykole oder Copolymere davon, z.B. aus Polyurethan und Polyethylenglykol, verwendet, um daraus Nanofasern durch Elektrospinnen zu erzeugen. In gleicher Weise bevorzugt sind anorganische Materialien, wie Oxide, bevorzugt Gläser, wie Quarz-, Silikat-, Alkalisilikat- oder Wasserglas, oder Metallalkoxy-Kondensate oder Kombinationen davon.

Es können Nanofasern auf die Oberfläche eines Trägermaterials aufgebracht werden, um dessen Eigenschaften, insbesondere hinsichtlich der Benetzungsfähigkeit von Flüssigkeiten, zu modifizieren. So kann eine hydrophobe Oberfläche, z.B. eine nicht-poröse Oberfläche, wie etwa das Testfeld eines Arrays, zur Erhöhung der Benetzungsfähigkeit mit hydrophilen Nanofasern beschichtet werden. Eine hydrophile Oberfläche im Sinne der vorliegenden Erfindung weist dabei vorzugsweise einen intrinsischen Kontaktwinkel <90° mit Wasser auf.

Als intrinsischer Kontaktwinkel wird der Kontaktwinkel auf einer ideal glatten Oberfläche bezeichnet, der als Maß für die durch chemische Gruppen bestimmte Oberflächenenergie dient, ohne jeglichen Einfluss der Oberflächengeometrie.

Beispielsweise kann durch Ablage von Nanofasern auf Oberflächen das Benetzungsverhalten dramatisch verändert werden. Während ein Wassertropfen auf reinen PMMA-Oberflächen einen Kontaktwinkel von ca. 70-80 Grad ausbildet, spreitet ein Wassertropfen auf einer PMMA-Oberfläche, die mit einer dünnen Schicht von Nanofasern aus Polyamid (PA) partiell bedeckt ist. Eine Menge von 10-500 mg/m², insbesondere von 50-300 mg/m², z.B. ca. 200 mg/m², Nanofasern z.B. aus Poly-εaminocaprolactam mit einer Dicke von 20-2000 nm, z.B. 600 nm, hat sich als günstig erwiesen.

Erfindungsgemäß erfolgt die Beschichtung einer Oberfläche, z.B. einer nicht-porösen Oberfläche, wie etwa ein Teststreifengehäuse, insbesondere im Bereich der Probenauftragzone, ein Bereich zwischen den Testfeldern eines Arrays, etc. mit hydrophoben Nanofasern, um die Benetzungsfähigkeit der Oberfläche zu verringern und eine Oberfläche mit hydrophoben oder superhydrophoben Eigenschaften zu erzeugen. Eine hydrophobe Oberfläche im Sinne der vorliegenden Erfindung weist dabei einen intrinsischen Kontaktwinkel ≥90° mit Wasser auf. Eine superhydrophobe Oberfläche im Sinne der vorliegenden Erfindung weist dabei vorzugsweise einen Kontaktwinkel ≥140°, vorzugsweise ≥150° mit Wasser auf.

Eine Nanofaserbeschichtung als Hydrophobsperre mit superhydrophoben Eigenschaften ist insbesondere von Interesse bei der Entwicklung von einem hygienischen Teststreifen, bei dem unbedingt verhindert werden muss, dass Probenflüssigkeit, z.B. Blut, auf dem Teststreifen haften bleibt. Um dies zu erreichen, wird aus einem hydrophoben Basismaterial, z.B. einem fluorierten oder teilfluorierten Polymer, wie etwa Polytetrafluorethylen (PTFE), einem modifizierten, z.B. löslichen PTFE, wie Teflon^{®} AF, einem Copolymer aus Tetrafluorethylen und Hexafluorpropylen (FEP), teilfluorierten Polyurethanen, fluorierten Polyaromaten etc. oder anderen oder einem Polyolefin, wie z.B. PP, Polypenten oder anderen oder Polyolefin-Copolymeren, ein Nanofasergebilde durch Elektrospinnen gefertigt. Es zeigte sich, dass bereits durch eine sehr dünne, z.B. 2 µm Schicht von solchen Fasermaterialien bzw. von Fasern mit Beads eine superhydrophobe Oberfläche geschaffen werden kann, auf der ein Wassertropfen einen Kontaktwinkel von ≥140° aufweist. Ein aufgesetzter Wassertropfen rollt auf einer solchen Oberfläche bereits bei einer Neigung von unter 20° ab. Ein Blutstropfen, der mit der Oberfläche einer solchen Schicht in Kontakt gebracht wird, zeigt keine Neigung, diese Schicht zu benetzen bzw. an ihr zu haften.

Ein besonders bevorzugter Gegenstand der Erfindung ist ein Testelement, umfassend (a) zumindest einen mit hydrophilen Nanofasern belegten Bereich und (b) zumindest einen mit hydrophoben Nanofasern belegten Bereich. Die mit hydrophilen Nanofasern belegten Bereiche sind vorzugsweise Testfelder, die zum Aufbringen von Probenflüssigkeiten, wie Blut, vorgesehen sind, um deren Benetzung zu verbessern. Die mit hydrophoben Nanofasern belegten Bereiche sind vorzugsweise in der Umgebung der Testfelder oder/und Probenaufgabestellen angeordnet, um eine unerwünschte Benetzung mit Probenflüssigkeit zu verhindern.

Es kann auch ein Gemisch von hydrophilen und hydrophoben Nanofasern auf eine Oberfläche. z.B. auf eine nicht-poröse Oberfläche, zur gleichmäßigeren Verteilung von Flüssigkeiten über die Oberfläche aufgebracht werden. Beispiele für Fasergemische sind Teflon^{®} AF und Poly(urethan-g-ethylenoxid).

Durch Aufbringen einer dünnen Schicht einer Mischung hydrophiler und hydrophober Nanofasern kann eine Oberfläche so modifiziert werden, dass sich ein aufgetragener Flüssigkeitstropfen, besonders ein wässriger Tropfen, gleichmäßig verteilt. Überraschenderweise wurde gefunden, dass beim Eintrocknen eines solchen Tropfens darin gelöste Stoffe eine wesentlich gleichmäßigere Schicht bilden als ohne die Anwesenheit von Nanofasern. Damit wird es möglich, eine in flüssiger Form aufgetragene Testchemie wesentlich gleichmäßiger zu verteilen, z.B. auf eine Elektrode eines elektrochemischen Sensors, als bisher. Dies ist auch von Bedeutung bei der Herstellung von Arrays, z.B. für molekulardiagnostische Untersuchungen. Besonders der Aspekt der Eigenfluoreszenz spielt hier eine große Rolle. Durch die extrem geringe Menge an Material, z.B. 10-500 mg/m² , die für den Effekt notwendig ist, ergibt sich nur eine sehr niedrige Eigenfluoreszenz, was wiederum das Signal/Rauschverhältnis bei der Auswertung der Arrays günstig beeinflusst.

Außerdem betrifft die Erfindung ein Verfahren zur qualitativen oder/und quantitativen Bestimmung eines Analyten in einer Probe, bei dem man ein Testelement wie zuvor beschrieben verwendet. Das Verfahren kann ein immunchemisches Verfahren oder ein auf Nukleinsäurehybridisierung beruhendes Verfahren oder auch ein enzymatisches Verfahren sein. Bevorzugte Anwendungen sind elektrochemische oder/und photometrische Nachweisverfahren, z.B. zum Nachweis von Glucose aus Blut oder anderen Körperflüssigkeiten.

Noch ein weiterer Gegenstand der Erfindung ist die Verwendung von Nanofasermaterial, als Filter in einem Testelement, das zum Nachweis von Analyten dient, wobei die Nanofasern elektrogesponnen sind, eine Länge von ≥ 2 mm aufweisen und Beads enthalten. Der Filter enthält die Nanofasern aufgebracht auf ein poröses Trägermaterial, wie zuvor angegeben.

Die Vorteile der Erfindung bestehen insbesondere darin, dass Vliese oder Filtermaterialien zur Verwendung auf Teststreifen erzeugt werden können, die wesentlich dünner sind, feinere gleichmäßigere Porenweite aufweisen und weniger Material erfordern als Materialien gemäß dem Stand der Technik. Von Vorteil ist auch, dass die Benetzbarkeit von Oberflächen durch Nanofasern dramatisch verbessert werden kann. Weiterhin von Vorteil ist, dass aus hydrophoben Polymeren superhydrophobe Oberflächen erzeugt werden können, auf denen ein Wassertropfen oder ein Blutstropfen sich nicht hält, sondern unter einem geringen Winkel bereits abgerollt wird. Vorteilhaft ist weiterhin, dass es bei der Separation von Blut praktisch nicht zur Hämolyse kommt. Durch Nanofasern kann die Applikation von Nachweisreagenzien auf Oberflächen wesentlich homogener erfolgen als bisher und die Eigenfluoreszenz kann reduziert werden.

## Patentansprüche

1. Testelement zum Nachweis eines Analyten, umfassend hydrophobe Nanofasern aufgebracht auf ein poröses oder nicht-poröses Trägermaterial, wobei die Nanofasern elektrogesponnen sind, eine Länge von ≥ 2 mm aufweisen und Beads enthalten, und wobei die mit Nanofasern beschichtete Oberfläche einen intrinsischen Kontaktwinkel ≥ 90° mit wasser aufweist.

2. Testelement nach Anspruch 1,
**dadurch gekennzeichnet,**
**dass** die Nanofasern kontinuierlich sind.

3. Testelement nach Anspruch 1 oder 2,
**dadurch gekennzeichnet,**
**dass** die Nanofasern einen Durchmesser von 10-500 nm aufweisen.

4. Testelement nach einem der Ansprüche 1 bis 3,
**dadurch gekennzeichnet,**
**dass** die hydrophoben Nanofasern eine Oberfläche mit superhydrophoben Eigenschaften bilden.

5. Testelement nach einem der Ansprüche 1 bis 4,
**dadurch gekennzeichnet,**
**dass** die Nanofasern aus Polymeren bestehen, ausgewählt aus Polyolefinen, Polyaromaten, fluorierten oder teilfluorierten Polymeren, Polyestern, Polyamiden, Polyurethanen und Kombinationen oder Copolymeren davon.

6. Testelement nach einem der Ansprüche 1 bis 5,
**dadurch gekennzeichnet,**
**dass** die Nanofasern in Form von Vliesen, Geweben, Membranen, Schichten oder Kombinationen davon vorliegen.

7. Testelement nach einem der Ansprüche 1 bis 6,
**dadurch gekennzeichnet,**
**dass** es einen Teststreifen umfasst.

8. Testelement nach einem der Ansprüche 1 bis 6,
**dadurch gekennzeichnet,**
**dass** es einen Testarray umfasst.

9. Testelement nach einem der Ansprüche 1 bis 8,
**dadurch gekennzeichnet,**
**dass** es Nanofasern aufgebracht auf ein poröses Trägermaterial ausgewählt aus Papieren, Vliesen und Membranen enthält.

10. Testelement nach Anspruch 9,
**dadurch gekennzeichnet,**
**dass** die Nanofasern auf zumindest eine Oberfläche eines porösen Trägermaterials aufgebracht sind.

11. Testelement nach Anspruch 9 oder 10,
**dadurch gekennzeichnet,**
**dass** es ein Filterelement zur Abtrennung von partikulären Bestandteilen aus einer Probe enthält, das zumindest teilweise aus Nanofasern besteht.

12. Testelement nach Anspruch 11,
**dadurch gekennzeichnet,**
**dass** das Filterelement ein Element zur Abtrennung von Blutzellen, vorzugsweise Erythrozyten, ist.

13. Testelement nach einem der Ansprüche 1 bis 8,
**dadurch gekennzeichnet;**
**dass** die Nanofasern auf die Oberfläche eines nicht-porösen Trägermaterials aufgebracht sind.

14. Testelement nach einem der Ansprüche 1 bis 13,
**dadurch gekennzeichnet,**
**dass** es eine mit hydrophoben Nanofasern beschichtete Oberfläche zur Verringerung der Benetzungsfähigkeit der Oberfläche umfasst.

15. Testelement nach Anspruch 14,
**dadurch gekennzeichnet,**
**dass** die beschichtete Oberfläche superhydrophobe Eigenschaften umfasst.

16. Testelement nach einem der Ansprüche 1 bis 15, umfassend
(a) zumindest einen mit hydrophilen Nanofasern belegten Bereich und
(b) zumindest einen mit hydrophoben Nanofasern belegten Bereich.

17. Testelement nach einem der Ansprüche 1 bis 16,
**dadurch gekennzeichnet,**
**dass** es eine mit einem Gemisch von hydrophilen und hydrophoben Nanofasern beschichtete Oberfläche zur gleichmäßigen Verteilung von Flüssigkeiten über die Oberfläche umfasst.

18. Verfahren zur qualitativen oder/und quantitativen Bestimmung eines Analyten in einer Probe,
**dadurch gekennzeichnet,**
**dass** man ein Testelement nach einem der Ansprüche 1 bis 17 verwendet.

19. Verwendung von Nanofasermaterial als Filter in einem Testelement zum Nachweis von Analyten, wobei die Nanofaserneine elektrogesponnen sind, eine Länge von ≥ 2 mm aufweisen und Beads enthalten, und wobei der Filter die Nanofasern aufgebracht auf ein poröses Trägermaterial enthält.

## Claims

1. A test element for detecting an analyte, comprising hydrophobic nanofibres applied to a porous or non-porous support material, wherein the nanofibres are electrospun, have a length of ≥ 2 mm and contain beads, and wherein the surface coated with nanofibres has an intrinsic contact angle of ≥ 90° with water.

2. A test element according to Claim 1,
**characterised in that** the nanofibres are continuous.

3. A test element according to Claim 1 or 2,
**characterised in that** the nanofibres have a diameter of 10 to 500 nm.

4. A test element according to any one of Claims 1 to 3,
**characterised in that** the hydrophobic nanofibres form a surface having superhydrophobic properties.

5. A test element according to any one of Claims 1 to 4,
**characterised in that** the nanofibres consist of polymers selected from polyolefins, polyaromatics, fluorinated or partially fluorinated polymers, polyesters, polyamides, polyurethanes and combinations or copolymers thereof.

6. A test element according to any one of Claims 1 to 5,
**characterised in that** the nanofibres are present in the form of fleeces, fabrics, membranes, layers or combinations thereof.

7. A test element according to any one of Claims 1 to 6,
**characterised in that** it comprises a test strip.

8. A test element according to any one of Claims 1 to 6,
**characterised in that** it comprises a test array.

9. A test element according to any one of Claims 1 to 8,
**characterised in that** it contains nanofibres applied to a porous support material selected from papers, fleeces and membranes

10. A test element according to Claim 9,
**characterised in that** the nanofibres are applied to at least one surface of a porous support material.

11. A test element according to Claim 9 or 10,
**characterised in that** it includes a filter element for separating particulate components from a sample, which filter element consists at least partly of nanofibres.

12. A test element according to Claim 11, **characterised in that** the filter element is an element for separating blood cells, preferably erythrocytes.

13. A test element according to any one of Claims 1 to 8,
**characterised in that** the nanofibres are applied to the surface of a non-porous support material.

14. A test element according to any one of Claims 1 to 13,
**characterised in that** it comprises a surface coated with hydrophobic nanofibres to reduce the wettability of the surface.

15. A test element according to Claim 14,
**characterised in that** the coated surface has superhydrophobic properties.

16. A test element according to any one of Claims 1 to 15, comprising
(a) at least one area covered with hydrophilic nanofibres and
(b) at least one area covered with hydrophobic nanofibres.

17. A test element according to any one of Claims 1 to 16,
**characterised in that** it comprises a surface coated with a mixture of hydrophilic and hydrophobic nanofibres for the uniform distribution of fluids over the surface.

18. A method for the qualitative or quantitative determination of an analyte in a sample,
**characterised in that** a test element according to any one of Claims 1 to 17 is used.

19. Use of nanofibre material as a filter in a test element for the detection of analytes, wherein the nanofibres are electrospun, have a length of ≥ 2 mm and contain beads, and wherein the filter contains the nanofibres applied to a porous support material.

## Revendications

1. Élément d'essai pour la détection d'un analyte, comprenant des nano fibres hydrophobes appliquées sur un matériau support poreux ou non poreux, sachant que les nano fibres sont filées par electrospinning, présentent une longueur ≥ 2 mm et contiennent des perles, et sachant que la surface revêtue de nano fibres présente un angle de contact intrinsèque ≥ 90° avec l'eau.

2. Élément d'essai selon la revendication 1, **caractérisé en ce que** les nano fibres sont continues.

3. Élément d'essai selon la revendication 1 ou 2, **caractérisé en ce que** les nano fibres présentent un diamètre de 10-500 nm.

4. Élément d'essai selon l'une quelconque des revendications 1 à 3, **caractérisé en ce que** les nano fibres hydrophobes forment une surface à propriétés super hydrophobes.

5. Élément d'essai selon l'une quelconque des revendications 1 à 4, **caractérisé en ce que** les nano fibres se composent de polymères, sélectionnés parmi les polyoléfines, les polyaromates, les polymères fluorés ou partiellement fluorés, les polyesters, les polyamides, les polyuréthanes et des combinaisons ou des copolymères de ces derniers.

6. Élément d'essai selon l'une quelconque des revendications 1 à 5, **caractérisé en ce que** les nano fibres sont présentes sous forme de non tissés, de tissus, de membranes, de couches ou de combinaisons de ces derniers.

7. Élément d'essai selon l'une quelconque des revendications 1 à 6, **caractérisé en ce qu'**il comprend une bande d'essai.

8. Élément d'essai selon l'une quelconque des revendications 1 à 6, **caractérisé en ce qu'**il comprend un réseau d'essai.

9. Élément d'essai selon l'une quelconque des revendications 1 à 8, **caractérisé en ce qu'**il contient des nano fibres appliquées sur un matériau support poreux sélectionné parmi les papiers, les non tissés et les membranes.

10. Élément d'essai selon la revendication 9, **caractérisé en ce que** les nano fibres sont appliquées sur tout au moins une surface d'un matériau support poreux.

11. Élément d'essai selon la revendication 9 ou 10, **caractérisé en ce qu'**il contient un élément de filtre pour la séparation de constituants particulaires hors d'un échantillon, qui se compose au moins partiellement de nano fibres.

12. Élément d'essai selon la revendication 11, **caractérisé en ce que** l'élément de filtre est un élément pour la séparation de cellules sanguines, de préférence d'érythrocytes.

13. Élément d'essai selon l'une quelconque des revendications 1 à 8, **caractérisé en ce que** les nano fibres sont appliquées sur la surface d'un matériau support non poreux.

14. Élément d'essai selon l'une quelconque des revendications 1 à 13, **caractérisé en ce qu'**il comprend une surface revêtue de nano fibres hydrophobes en vue de la diminution du pouvoir mouillant de la surface.

15. Élément d'essai selon la revendication 14, **caractérisé en ce que** la surface revêtue possède des propriétés super hydrophobes.

16. Élément d'essai selon l'une quelconque des revendications 1 à 15, comportant
(a) au moins un domaine recouvert de nano fibres hydrophiles et
(b) au moins un domaine recouvert de nano fibres hydrophobes.

17. Élément d'essai selon l'une quelconque des revendications 1 à 16, **caractérisé en ce qu'**il comporte une surface revêtue d'un mélange de nano fibres hydrophiles et hydrophobes en vue d'une répartition uniforme de liquides sur la surface.

18. Procédé en vue de la détermination qualitative et/ou quantitative d'un analyte dans un échantillon, **caractérisé en ce que** l'on utilise un élément d'essai selon l'une quelconque des revendications 1 à 17.

19. Utilisation de matériel à nano fibres dans un élément d'essai pour la détection d'analytes, sachant que les nano fibres sont filées par electrospinning, présentent une longueur ≥ 2 mm et contiennent des perles, et sachant que le filtre contient les nano fibres appliquées sur un matériau support poreux.
